# EUROPEAN PATENT APPLICATION

(11) **EP 1 224 936 A1**
(43) Date of publication of application: **24.07.2002**
(21) Application number: 00961099.9
(22) Date of filing: 20.09.2000
(51) Int. Cl.: A61K 31/765, A61P 3/00, A61P 43/00

(54) **PHYSICAL STRENGTH ENHANCING AGENTS AND GLYCOGEN ACCUMULATION PROMOTING AGENTS**

(30) Priority: 20.09.1999 JP 26575599; 14.07.2000 JP 2000214529
(71) Applicant: AMATO PHARMACEUTICAL PRODUCTS, LTD., Fukuchiyama-shi, Kyoto 620-0932 (JP)
(72) Inventor: TAKADA, Shigeo, Isehara-shi, Kanagawa 259-1112 (JP); NAGATO, Yasukazu, Atsugi-shi, Kanagawa 243-0122 (JP); YAMAMURA, Masaichi, Atsugi-shi, Kanagawa 243-0122 (JP); MURAKAMI, Masahiro, Osaka 547-0026 (JP); IWAGAKI, Suketsune, Hadano-shi, Kanagawa 257-0028 (JP); ARAI, Toshihiro, Atsugi-shi, Kanagawa 243-0804 (JP); TERAO, Tamotsu, Naka-gun, Kanagawa 259-0112 (JP)
(74) Representative: Sternagel, Fleischer, Godemeyer & Partner Patentanwälte
(86) International application number: JP0006400
(87) International publication number: WO0121182

(57) **Abstract**

The objective of the present invention is to provide an agent for enhancing stamina which is effective in improving the exercise ability of sports enthusiasts, athletes or the like who wish to improve their own endurance while exercising, and a substance which can increase the accumulation amount of glycogen in the liver and/or muscle. The present invention provides an agent for enhancing stamina which comprises a mixture of cyclic and/or straight chain poly lactic acids having a condensation degree of 3 to 20, and an agent for promoting glycogen accumulation which comprises a mixture of cyclic and/or straight chain poly lactic acids having a condensation degree of 3 to 20.

## Description

### TECHNICAL FIELD

The present invention relates to an agent for enhancing stamina and an agent for promoting glycogen accumulation that are useful as a supplement for athletes. More specifically, the present invention relates to an agent for enhancing stamina and an agent for promoting glycogen accumulation that comprise, as an active ingredient, a mixture of poly lactic acids having certain condensation degree.

### BACKGROUND ART

Recently in the fields of exercise physiology, nutritional physiology and the like, dietary guidance is being given to various types of athletes in order to improve stamina including endurance, muscle power, and exercise ability. For example, protein is necessary for muscle strengthening, and fat and carbohydrates are important energy sources. Further, calcium ingestion is necessary for bone strengthening, and iron, which is a component of hemoglobin, plays a very important role in in vivo transportation of oxygen. For example, it has been reported that energy production ability is improved by a high fat diet, enabling promotion of establishment of efficient energy metabolism, and in particular the effectiveness of monovalent unsaturated fatty acid has been reported. It has also been reported that high exercise ability can be obtained when a high protein diet is ingested.

Also, it has become known in recent years that various ingredients are involved in the regulation of immunocompetence and metabolic function. These ingredients are now used in various nutrient preparations. For example, arginine, an ingredient which is a semi-essential amino acid for infants, is known to be necessary for detoxicating toxic ammonia which is produced when protein is metabolized in vivo, and is also known to function as a precursor of polyamine, to participate in muscle metabolism, to have an effect of improving nitrogen availability efficiency in vivo, to have an immuno activating action and the like.

Moreover, glutamine, an amino acid that constitutes 50 to 60% of the skeletal muscle amino acid pool and approximately 20% of the plasma amino acid pool, is said to be a major energy source in small intestine epithelium cells. Also, glutamine deficiency is thought to be a cause of contracted bowel, and is also suggested to have an effect on the immune system. For these reasons, glutamine has recently been the subject of attention in the areas of enteral feeding agents and transfusion, and technology concerning the application of glutamine has been disclosed (Japanese Patent Application Laying-Open (Kokai) Nos. 2-119762, 3-264525, and 5-236909). Further, glutamine is also noted in the field of sports physiology and has been shown to have an effect on supplementation of glycogen consumed by exercise and recovery of immunocompetence at the time of fatigue (Tadao YOSHIDA, Food Chemical Journal, 1994-10, 46).

Further, as agents for enhancing stamina and agents for recovery from fatigue, there are many health foods on the market, which use a variety of plant and animal extracts and so on like folk medicine, but few of them are ingested daily in large quantities and the safety of such health foods when ingested in large quantities for a long period has not been confirmed.

As a medicament, there are used a medicament comprising a specified active ingredient which is highly concentrated, and a medicament comprising a specified active ingredient which is chemically synthesized. The therapeutic effect of such a medicament clearly appears, but here may be a risk of side effects. In the case of health foods, supplements and the like, safety concerning ingestion over a long period of time should take priority over a risk of side effects.

Glycogen is one type of glucan which is a homopolysaccharide comprising glucose, and is widely distributed in animals as storage polysaccharide in a granular state in most cells, and in particular it is richly present in the liver and muscle. While glycogen in the liver is an energy source for an organism, glycogen in the muscle is an energy source for muscle contraction. The roles of these two glycogens are different.

The biosynthetic pathways of glycogen start from glucose. Glucose is converted to UDP glucose via glucose 6-phosphate and glucose 1-phosphate. Next, the UDP-glucose is incorporated into the primer of glycogen by glycogen synthase. Sugar chains are elongated by repetition of these steps, and branch formation of α1→6 linkage is performed with α-1,4-glucan branching enzyme.

On the other hand, in the metabolic pathways of glycogen, glucose 1-phosphate is first produced from glycogen by glucose phosphorylase. In the liver, glycogen is converted to glucose via glucose 6-phosphate, and then the glucose is released into blood. Further, in the muscle and other tissues, glucose 6-phosphate is converted into fructose 6-phosphate which enters the glycolysis system, or glucose 6-phosphate also enters the pentose phosphate cycle.

As described above, the degradation product from glycogen will be the energy source of each organ. Therefore, from various points of view including recovery from fatigue and improvement in exercise ability, it is desirable to increase the accumulation amount of glycogen in the liver and/or muscle.

Therefore, there is a need to develop a medicament which is useful in promoting glycogen accumulation in the liver and/or muscle.

It has been reported in the research up to now that a mixture of cyclic and/or straight chain poly L-lactic acids having a condensation degree of 3 to 20 is useful as an antineoplastic agent (Japanese Patent Application Laying-Open (Kokai) Nos. 9-227388 and 10-130153) and also as a QOL improving agent for cancer patients (Japanese Patent Application No. 11-39894 Specification; Bulletin of Japan Society of Clinical Oncology, Vol. 33, No. 3, p. 493). However, the effect of a mixture of cyclic and/or straight chain poly L-lactic acids having a condensation degree of 3 to 20 on athletes has not been reported. Particularly, whether a mixture of cyclic and/or straight chain poly L-lactic acids having a condensation degree of 3 to 20 can contribute to raising the endurance of athletes, that is, their stamina and endurance while exercising, and whether it can increase the accumulation amount of glycogen in the liver and/or muscle, remain totally unknown.

### DISCLOSURE OF THE INVENTION

An objective of the present invention is to provide an agent for enhancing stamina which is useful for improving the exercise ability of sports enthusiasts, athletes or the like who wish to improve their own endurance while exercising; and a supplement using the above agent for enhancing stamina.

Another objective of the present invention is to provide a substance that can increase the accumulation amount of glycogen in the liver and/or muscle; and a medicament or supplement comprising the substance.

Still another objective of the present invention is to provide an agent for enhancing stamina and an agent for promoting glycogen accumulation which possess high biocompatibility and are made of relatively low cost raw material.

In order to achieve the aforementioned objects, the present inventors have administered a mixture of cyclic and/or straight chain poly lactic acids having a condensation degree of 3 to 20 to long-distance runners (students) for a certain period, and measured the training load, weight fluctuation and some physiological parameters of each runner. As a result, the present inventors have found that runners administered with a mixture of poly lactic acids showed improved resistance to endurance training and improved exercise records. Further, the present inventors have found that administering cyclic lactic acid oligomers to mice significantly increase the accumulation amounts of glycogen in the muscle and liver. The present invention has been completed based on these findings.

Thus, according to the present invention, there is provided an agent for enhancing stamina that comprises a mixture of cyclic and/or straight chain poly lactic acids having a condensation degree of 3 to 20.

The agent for enhancing stamina of the present invention is used, for example, to maintain or improve the endurance of athletes, or to recover from fatigue.

According to another aspect of the present invention, there is provided an agent for promoting glycogen accumulation that comprises a mixture of cyclic and/or straight chain poly lactic acids having a condensation degree of 3 to 20.

The agent for promoting glycogen accumulation of the present invention is used, for example, to recover from fatigue, enhance the exercise ability of the muscle or improve the QOL of patients, or is used for improving carnosity.

The mixture of cyclic and/or straight chain poly lactic acids having a condensation degree of 3 to 20 used in the present invention preferably comprises a cyclic lactic acid oligomer which is shown by the following formula (1): 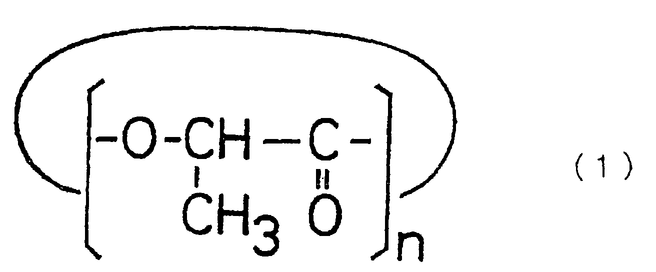 wherein n represents an integer from 3 to 20.

Preferably, the lactic acid that is a repeating unit in the poly lactic acid, substantially consists of L-lactic acid.

The mixture of cyclic and/or straight chain poly lactic acids having a condensation degree of 3 to 20 used in the present invention is, for example, a fraction obtained by condensing lactic acids by dehydration under an inactive atmosphere, subjecting the ethanol- and methanol-soluble fraction of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3. The condensation by dehydration is preferably performed by stepwise decompression and temperature rise under nitrogen gas atmosphere. The reverse phase column chromatography is preferably performed by ODS column chromatography.

It is preferred that the agent for enhancing stamina and the agent for promoting glycogen accumulation according to the present invention do not substantially comprise a chain lactic acid oligomer having a condensation degree of 3 to 20.

According to still another aspect of the present invention, there is provided a health food or a supplement which comprises the agent for enhancing stamina and the agent for promoting glycogen accumulation according to the present invention as mentioned above.

According to still another aspect of the present invention, there is provided a use of a mixture of cyclic and/or straight chain poly lactic acids having a condensation degree of 3 to 20, in the production of the agent for enhancing stamina, the agent for promoting glycogen accumulation, or the supplement comprising it.

According to still another aspect of the present invention, there is provided a method for enhancing stamina or a method for promoting glycogen accumulation, which comprises administering an effective amount of a mixture of cyclic and/or straight chain poly lactic acids having a condensation degree of 3 to 20 to mammal such as human.

### BRIEF DESCRIPTION OF THE DRAWINGS

Figure 1 shows a method for conducting the tests in the present examples.
Figure 2 is a graph showing training load and weight fluctuation for 3 weeks in which CPL was administered.
Figure 3 is a graph showing diurnal weight fluctuation.
Figure 4 is a graph showing the changes in plasma lipid after 30 days of training camp.
Figure 5 is a graph showing the changes in erythrocyte lipid.
Figure 6 shows a plasma particle image.
Figure 7 is a graph showing the comparison of plasma particle in the control group with that in the group administered with CPL.
Figure 8 is a graph showing the proportion (%) of NK cells obtained using CD56.
Figure 9 is a graph showing changes with time in energy consumption during exercise and during recovery period.
Figure 10 is a graph showing changes with time in respiratory exchange ratio during exercise and during recovery period.
Figure 11 is a graph showing changes in energy consumption before and after administration of CPL.
Figure 12 is a graph showing changes in blood lactic acid level after time trial.
Figure 13 is a graph showing changes in energy consumption before and after administration of CPL.
Figure 14 shows a mass spectrum of the mixture of poly lactic acids obtained by Production Example 1 of the present specification.

### THE BEST MODE FOR CARRYING OUT THE INVENTION

The embodiments and methods for carrying out the present invention are described in detail below.

The agent for enhancing stamina and the agent for promoting glycogen accumulation of the present invention comprises, as an active ingredient, a mixture of cyclic and/or straight chain poly lactic acids having a condensation degree of 3 to 20.

Preferably, the agent for enhancing stamina of the present invention can be widely used for maintaining or improving the endurance of athletes, or for recovering from fatigue generally.

Particularly preferably, the agent for enhancing stamina of the present invention can exert its effect by being administered to athletes who need endurance, such as long-distance runners. The endurance-training load for a long-distance runner is, for example, 21 km/day, and 40 to 60 km/day during 2 months of a training camp. A crucial problem for a long-distance runner is whether the runner can withstand such training stress.

For example, weight fluctuation is given as one index of resistance to training stress. That is, the amount of weight fluctuation can function as an index because less weight reduction is observed in a runner with a high level of endurance (10,000 m).

Ingestion of the agent for enhancing stamina of the present invention comprising a mixture of cyclic and/or straight chain poly lactic acids having a condensation degree of 3 to 20 leads to less weight reduction and immediate weight recovery during a training period. Therefore, it can be considered that the agent for enhancing stamina of the present invention is useful in maintaining or improving the endurance of athletes.

Further, ingestion of the agent for enhancing stamina not only causes less weight reduction and rapid weight recovery, but also it was found that it causes a significant increase in the number of natural killer (NK) cells, revealing that the immune system is activated by the agent. These results indicate that the agent for enhancing stamina of the present invention can be used for recovery from fatigue, particularly for recovery from fatigue in the case of athletes.

Specific examples of the possible uses of the agent for promoting glycogen accumulation of the present invention are described in the following (1) to (4). However, the following examples of use are given for merely exemplary purposes, and are not intended to limit the intended purposes of the agent for promoting glycogen accumulation of the present invention as far as this agent is used to promote glycogen accumulation.
(1) One reason for fatigue is depletion of glycogen in the muscle and/or liver. The accumulation amount of glycogen in vivo can be increased by daily ingestion of the agent for promoting glycogen accumulation of the present invention as, for example, a drinkable preparation, as a result of which fatigue can be alleviated or restored. That is, ingestion of the agent for promoting glycogen accumulation of the present invention enables an increase in the time of work without feeling fatigue, and can also achieve alleviation of reduction in efficiency as well as prevention of accidents caused by fatigue. Therefore, the agent for promoting glycogen accumulation of the present invention is useful for recovering from fatigue.
(2) Increasing the storage amount of glycogen in the muscle is essential for athletes to improve their performance. Although various methods have been proposed for increasing the accumulation amount of glycogen in the muscle, such increase have been generally difficult. The accumulation amount of glycogen in the muscle can be easily, safely, and efficiently increased by ingesting the agent for promoting glycogen accumulation of the present invention, more preferably by the combination of ingestion of the agent and exercise, thereby contributing to improving the performance of athletes. In particular, a cyclic lactic acid oligomer which is an active ingredient of the agent for promoting glycogen accumulation of the present invention comprises lactic acids having high bioavailability as an ingredient, and therefore the agent is characterized in that it can be safely used without being classified as a forbidden drug. Thus, the agent for promoting glycogen accumulation of the present invention is useful for enhancing the exercise ability of the muscle.
(3) Interleukin 6 is known as one of the substances which cause cachexia in cancer (particularly, terminal cancer) patients. Interleukin 6 significantly decreases the glycogen level in the liver, thereby lowering the QOL (Quality of Life) of patients. Administration of the agent for promoting glycogen accumulation of the present invention to such patients makes it possible to increase the glycogen accumulation level in the liver, or at least to suppress reduction of the glycogen level in the liver, so that the QOL of patients can be improved. Thus, the agent for promoting glycogen accumulation of the present invention is useful for improving the QOL of patients.
(4) Fresh seafood and livestock products are generally known to be tasty because of their rich content of glycogen. The use of the agent for promoting glycogen accumulation of the present invention can improve the carnosity of cultured seafood and livestock products by increasing the glycogen content of the cultured seafood and livestock products to thereby achieve improved taste. Thus, the agent for promoting glycogen accumulation of the present invention is useful in improving carnosity.

In the agent for enhancing stamina, agent for promoting glycogen accumulation, and supplement comprising these according to the present invention, a mixture of cyclic and/or straight chain poly lactic acids having a condensation degree of 3 to 20 is used as an active ingredient.

The term "a mixture of poly lactic acids" used in the present invention means a mixture wherein cyclic and/or straight chain poly lactic acids having a condensation degree of 3 to 20 are present at any ratio. That is to say, the term "mixture" does not only mean a mixture of poly lactic acids having any condensation degree ranging from 3 to 20, but is also used as a concept including a mixture of cyclic and straight chain poly lactic acids. As is described below in the present specification, "a mixture of poly lactic acids" can be obtained by condensing lactic acids by dehydration and then performing purification by a suitable method. Although the term "a mixture of poly lactic acids" is used in the present specification for the sake of convenience, this term also includes a poly lactic acid consisting of a single ingredient such as a cyclic poly lactic acid having single condensation degree or a straight chain poly lactic acid having single condensation degree.

The term "condensation degree" is used to mean the number of lactic acid unit which is a repeating unit in poly lactic acids. For example, the cyclic poly lactic acid is assumed to have the following structural formula wherein n represents condensation degree (n = 3 to 20).

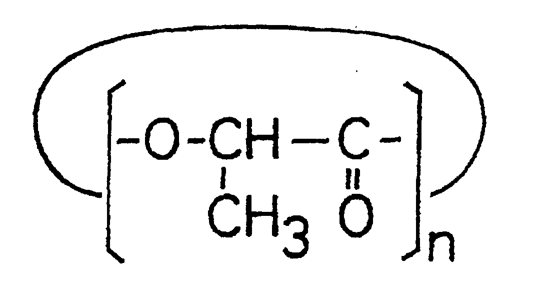

When "lactic acid" is simply referred to in the present specification, this lactic acid includes all of L-lactic acid, D-lactic acid or a mixture comprising these types of lactic acid at any ratio. Preferably in the present invention, the lactic acid substantially consists of L-lactic acid. The term "substantially" is used herein to mean that the ratio of L-lactic acid units in a mixture of poly lactic acids (number of L-lactic acid unit / number of L-lactic acid unit + number of D-lactic acid unit × 100) is, for example, 70% or more, preferably 80% or more, more preferably 85% or more, further more preferably 90% or more, and particularly preferably 95% or more. The ratio of L-lactic acid units in a mixture of poly lactic acids depends on the ratio of L-lactic acid and D-lactic acid that exist in lactic acids used as a starting substance.

The agent for enhancing stamina and the agent for promoting glycogen accumulation of the present invention are characterized by comprising a mixture of cyclic and/or straight chain poly lactic acids having a condensation degree of 3 to 20, preferably comprising at least a mixture of cyclic poly lactic acids. When the agent comprises a mixture of straight chain poly lactic acids, its content is not specifically limited, but is, of the whole mixture of poly lactic acids, preferably 50 weight % or less, more preferably 40 weight % or less, further more preferably 30 weight % or less, still further more preferably 20 weight % or less, ranging from, for example, 10 weight % or more to 20 weight %.

The agent for enhancing stamina and the agent for promoting glycogen accumulation of the present invention may not substantially comprise a chain lactic acid oligomer. The term "not substantially comprise" is used in the present specification to mean that the content of a mixture of straight chain poly lactic acids is, of the whole mixture of poly lactic acids, less than 10 weight %, more preferably less than 5 weight %, and further more preferably less than 3 weight %.

The methods for producing a mixture of cyclic and/or straight chain poly lactic acids having a condensation degree of 3 to 20 are not particularly limited, and the mixture of poly lactic acids can be obtained by the production methods described, for example, in Japanese Patent Application Laying-Open (Kokai) Nos. 9-227388 and 10-130153 or Japanese Patent Application No. 11-39894 (All publications cited herein are incorporated herein by reference in their entirety).

More specifically, for example, a mixture of cyclic and/or straight chain poly lactic acids having a condensation degree of 3 to 20 can be obtained by the following method A.

### Method A:

First, lactic acid (preferably, lactic acid substantially consisting of L-lactic acid) is condensed by dehydration under an inactive atmosphere. Examples of the inactive atmosphere include nitrogen gas and argon gas, and nitrogen gas is preferred.

Dehydration and condensation reaction is carried out at a temperature of 110°C to 210°C, preferably 130°C to 190°C under normal pressure to reduced pressure of approximately 1mmHg, and particularly preferably the reaction is carried out by stepwise decompression and stepwise temperature rise. A reaction period can be determined as appropriate. For example, the reaction can be carried out for 1 to 20 hours. Where stepwise decompression and stepwise temperature rise are applied, reaction is performed by dividing the reaction period into two or more partial reaction periods, and then determining pressure and temperature for each of the reaction periods. Where stepwise decompression is applied, pressure can be reduced, for example, from a normal pressure to 150mmHg and then to 3mmHg. Where stepwise temperature rise is applied, temperature can be raised, for example, from 145°C to 155°C and then to 185°C. Practically, the reaction can be carried out by using these conditions in combination, for example, 145°C, normal pressure, 3 hours; 145°C, 150mmHg, 3 hours; 155°C, 3mmHg, 3 hours; and 185°C, 3mmHg, 1.5 hours.

Subsequently, ethanol and methanol are added to the reaction mixture obtained by the dehydration and condensation reaction, and the mixture is filtered. The obtained filtrate is dried to obtain ethanol- and methanol-soluble fractions. The term "ethanol- and methanol-soluble fractions" is used in the present specification to mean fractions soluble in a mixed solution of ethanol and methanol. In order to obtain ethanol and methanol-soluble fractions, a reaction mixture obtained by dehydration and condensation reaction is mixed with ethanol and methanol, where the ratio of ethanol and methanol can be determined as appropriate. For example, the ratio is ethanol:methanol = 1 : 9. The order, method and the like for adding ethanol and methanol to a reaction mixture are not limited, and may be selected as appropriate. For example, ethanol may be added at first to the reaction mixture obtained by the dehydration and condensation reaction, and then methanol may be added thereto.

The thus obtained ethanol- and methanol-soluble fractions are subjected to reverse phase column chromatography, especially to chromatography where an octadecylsilane (ODS) column is used. First, fractions eluted with 25 to 50 weight % acetonitrile aqueous solution of pH 2 to 3 are removed, and then fractions eluted with 90 weight % or more acetonitrile aqueous solution of pH 2 to 3, preferably 99 weight % or more acetonitrile aqueous solution, are collected so as to obtain a mixture of cyclic and/or straight chain poly lactic acids having a condensation degree of 3 to 20.

The thus obtained mixture of cyclic and/or straight chain poly lactic acids is neutralized with an alkaline substance such as sodium hydroxide, and is dried under reduced pressure, and then according to standard techniques, the mixture can be formulated in a desired form as mentioned below.

Other examples of the methods for producing a mixture of cyclic and/or straight chain poly lactic acids having a condensation degree of 3 to 20 used in the present invention include a method described in Japanese Patent Application No. 11-265715 (hereinafter referred to as method B), or a method described in Japanese Patent Application No. 11-265732 (hereinafter referred to as method C) (All publications cited herein are incorporated herein by reference in their entirety). Methods B and C will be described specifically below.

### Method B:

Method B is a method for producing a cyclic lactic acid oligomer which comprises polymerizing lactid (3,6-dimethyl-1,4-dioxane-2,5-dione) in the presence of an alkali metal compound represented by RYMe [wherein R represents an aliphatic group, aromatic group, substituted or unsubstituted silyl group, or lactamide group (-CH(CH₃)CONH₂ group), Y represents oxygen atom, sulfur atom, or NR', in which R' represents hydrogen atom, aliphatic group or aromatic group, and Me represents alkali metal].

An aliphatic carbohydrate group in the present specification may be straight chain, branched-chain, cyclic, or any combination of these, and may be a saturated or unsaturated group. The carbon number of the aliphatic carbohydrate group is 1 to 12, preferably 1 to 6. Examples of the aliphatic carbohydrate group include a chain (including both straight chain and branched-chain) alkyl group such as methyl, ethyl, propyl, butyl, octyl and dodecyl; and a cycloalkyl group (for example, cyclopropyl, cyclobutyl, cyclopentyl, and cyclohexyl).

An aromatic carbohydrate group in the present specification includes an aryl group which may have a substituted group such as an alkyl group, and an arylalkyl group, and the carbon number thereof is 6 to 12, preferably 6 to 10. Examples of the aryl group which may have a substituted group such as an alkyl group include phenyl, tolyl, and naphthyl. Examples of the arylalkyl group include benzyl, phenethyl, and naphthylmethyl.

Examples of a substituted group of a substituted or unsubstituted silyl group include an aliphatic carbohydrate group or aromatic carbohydrate group. Specific examples of the substituted silyl group include a trimethylsilyl group, triphenylsilyl group or t-butyldimethylsilyl group.

Examples of alkali metal represented by Me include lithium, sodium or potassium, and lithium is preferred.

An alkali metal compound represented by RYMe can be obtained by allowing alkyl-alkali metal, such as n-butyl lithium, to react with R'-YH (wherein R' represents an aliphatic carbohydrate group or aromatic carbohydrate group, and Y represents oxygen atom or sulfur atom).

Specifically, reaction can be carried out by preparing a solution by dissolving an alcohol compound or thiol compound represented by R'-YH in an appropriate solvent (for example, an ether-based solvent, such as anhydrous tetrahydrofuran or anhydrous diethyl ether), adding alkyl-alkali metal, such as n-butyl lithium, to the solution in an amount almost equivalent to that of the alcohol compound or thiol compound, and then stirring the solution.

Reaction may be carried out at a low temperature (for example, -78°C) for several minutes to 1 hour.

When a cyclic lactic acid oligomer used in the present invention is produced by allowing lactid (3,6-dimethyl-1,4-dioxane-2,5-dione) to react in the presence of an alkali metal compound (RYMe), the cyclic lactic acid oligomer can be produced by adding a lactid solution in an appropriate solvent (for example, anhydrous tetrahydrofuran) to a reaction mixture containing the alkali metal compound obtained as described above, and stirring the solution.

The molar ratio of the amounts of the alkali metal compound (RYMe) and lactid is 1:1 to 1:10, preferably about 1:2 to 1:5, for example, 1:3 or 1:4.

A reaction temperature range is -78°C to room temperature. Reaction is preferably carried out by starting from a temperature of -78°C, and gradually raising it to room temperature. In addition, a reaction pressure is not specifically limited, and normal pressure is preferred.

As described above, the reaction is preferably carried out in the presence of a solvent. A preferred reaction solvent is a solvent inactive in reaction. For example, an ether-based solvent (anhydrous tetrahydrofuran, anhydrous diethylether, or the like) can be used. Reaction is preferably performed under an inactive gas atmosphere such as nitrogen gas and argon gas.

The mechanism of the above-described reaction for the synthesis of the cyclic lactic acid oligomer used in the present invention is described further below. However, the present invention is not restricted by this theory, and a cyclic lactic acid oligomer which is synthesized in a reaction with a mechanism differing from the mechanism below may be used in the present invention.

In the above reaction (herein below, an example is described wherein alkali metal is Li), first a lithium compound is reacted with lactid so that a derivative of chain lactic acids which is represented by the following formula is synthesized: 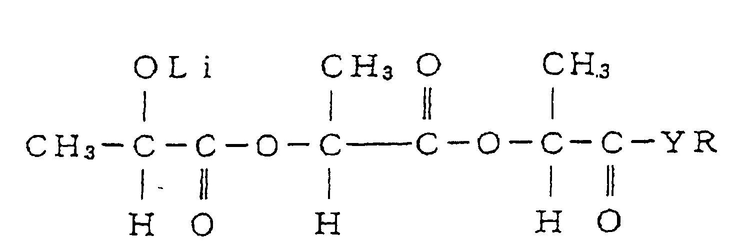 (wherein Y and R have the same meaning as described above) .
Then, lactid is reacted with this compound, so that a chain lactic acid oligomer which is represented by the following formula is synthesized: 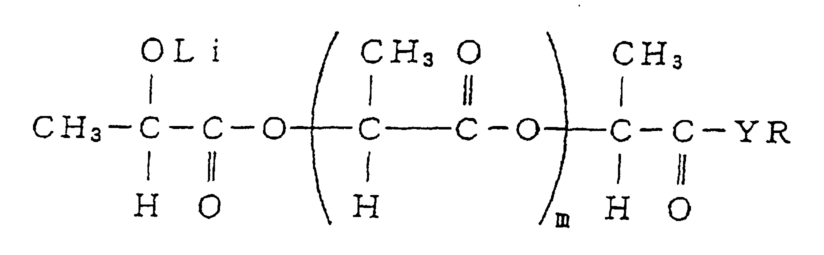 (wherein m represents a number from 1 to 21, and Y and R have the same meaning as described above). Then RYLi leaves from the compound, which is followed by cyclization. Thus the cyclic lactic acid oligomer of the above formula (1) is synthesized.

The composition (that is, the mixing ratio of a cyclic lactic acid oligomer and a chain lactic acid oligomer) of the lactic acid oligomer obtained as described above fluctuates depending on the alkali metal compound used as a reaction assistant. Where an alkali metal compound of alkyl alcohol having a carbon number of 1 to 3 (ROMe) (wherein R represents an alkyl group with carbon number 1 to 3, and Me represents alkali metal) is used as an alkali metal compound, a mixture of a cyclic lactic acid oligomer and a chain oligomer (proportion of the cyclic lactic acid oligomer: 80 to 85 weight %) is obtained. When a lithium compound of alkyl alcohol having a carbon number of 4 or more such as t-butyl alcohol, or thiophenol compound is used as an alkali metal compound, substantially only a cyclic lactic acid oligomer can be selectively obtained.

The polymerization degree of the cyclic lactic acid oligomer used in the present invention is 3 to 20, preferably 3 to 17. This polymerization degree fluctuates depending on the type of alkali metal compound to be used, reaction temperature and reaction period.

Further, a mixture of cyclic (and chain, depending on the case) lactic acid oligomers with different polymerization degrees are thought to be present in the reaction product resulting from polymerization reaction of lactid in the presence of the above alkali metal compound. In the present invention, a mixture comprising lactic acid oligomers with different polymerization degrees can be used. However, a single lactic acid oligomer having a single polymerization degree may be obtained by purifying with a technique (for example, gel filtration, HPLC or the like) appropriate for separating compounds of differing molecular weights from a reaction mixture containing the above lactic acid oligomers with different polymerization degrees, and may be used herein.

In the above method of producing a cyclic lactic acid oligomer, substantially only cyclic lactic acid oligomers can also be selectively obtained by carrying out reaction in the same manner as described above with the exception that an alkali metal compound of lactamide (in particular, a lithium compound) (that is, a compound wherein R represents -CH(CH₃)CONH₂ group) is used as an alkali metal compound.

### Method C:

This method comprises:
(i) a first heating step which comprises heating lactic acid under a pressure condition of 350 to 400 mmHg and to a temperature of 120 to 140°C so as to perform dehydration and condensation, and distilling off and removing only by-product water without distilling lactid off;
(ii) a second heating step for synthesizing a product condensed by dehydration comprising chain lactic acid oligomers as the main ingredient, which comprises, after completion of the first heating step, heating the reaction product to a temperature of 150 to 160°C while reducing the reaction pressure to 15 to 20 mmHg at a decompression rate of 0.5 to 1 mmHg/min, wherein only by-product water is distilled off and removed while avoiding distillation of lactid; and after the reaction pressure is reduced to 15 to 20 mmHg, maintaining the reaction under the same pressure condition and at a reaction temperature of 150 to 160°C;
(iii) a third heating step for synthesizing cyclic oligomers which comprises, after completion of the second heating step, heating under a pressure condition of 0.1 to 3 mmHg and at 150 to 160°C to cyclize the chain lactic oligomer.

In this method, first, in the first heating step, lactic acid is heated under reduced pressure to perform dehydration and compression reaction. In this case the reaction period is 3 to 12 hours, preferably 5 to 6 hours. To allow the reaction in the first heating step to proceed smoothly, by-product water produced by condensation of lactic acids by dehydration is distilled off. At this time, distillation of by-product water is performed such that lactid, which is the dehydrated condensed product of two molecules of lactic acid, is not distilled off. To achieve such purpose, the reaction pressure is maintained at a reduced pressure, preferably 300 to 500 mmHg, more preferably 350 to 400 mmHg. Under this pressure condition, heating is performed at a temperature range of 100 to 140°C, preferably 130 to 140°C. The reaction product produced by reaction in the first heating step mainly comprises as the main ingredient a dehydrated condensed product of 3 to 23 molecules of lactic acid.

To obtain oligomers having an increased average degree of polymerization in the second heating step after completion of the above first heating step, heating is performed at a temperature higher than the reaction temperature of the above first heating step, preferably at 145°C to 180°C, more preferably 150°C to 160°C, while the reaction pressure is reduced to 10 to 50 mmHg, preferably 15 to 20 mmHg, so that dehydration and condensation reaction is further continued.

As with the reaction in the above first heating step, reaction is performed under a condition where by-product water, but not lactid, is distilled off, to allow the reaction to proceed smoothly. The rate at which reaction pressure is reduced to a pressure in the above range (decompression rate) is normally required to be maintained within a range of 0.25 to 5 mmHg/min, preferably 0.5 to 1 mmHg/min, in order to avoid distillation of lactid and increase the reaction efficiency. A decompression rate lower than the above range is not preferred because it will increase the time required to reduce pressure to a given pressure. On the other hand, a decompression rate higher than the above range is also not preferred because it will cause lactid to be distilled off together with by-product water.

After the reaction pressure is reduced to a certain pressure, reaction is further continued at that reaction pressure. The heating time period in this case is 3 to 12 hours, preferably 5 to 6 hours.

A lactic acid oligomer having an average polymerization degree of 3 to 30, preferably 3 to 23 is obtained by the reaction in the above second heating step. The proportion of cyclic oligomers in the oligomers in this case is normally about 70 to 80 weight %.

In the third heating step, after completion of the above second heating step, a reaction pressure is maintained at 0.25 to 5 mmHg, preferably 0.5 to 1 mmHg, and reaction is further continued at a temperature of 145 to 180°C, preferably 150 to 160°C. A reaction period is 3 to 12 hours, preferably 5 to 6 hours. By-product water produced in this case is also distilled off. In this case, distillation of lactid is preferably avoided. However, since the reaction product contains almost no lactid, it is not required to specially lower the decompression rate.

Lactic acid oligomers produced by reaction in the above third heating step have an average polymerization degree of 3 to 30, preferably 3 to 23, and contain cyclic oligomer in the proportion of 90 weight % or more, preferably 99 weight % or more.

The above methods A, B and C merely show some of specific examples of methods of producing a mixture of poly lactic acids used in the present invention. A mixture of poly lactic acids which is produced by other methods can also be used in the present invention.

The dosage form of the agent for enhancing stamina and the agent for promoting glycogen accumulation of the present invention is not particularly limited, and any form suitable for the purpose can be selected from dosage forms for oral or parenteral administration. Dosage forms for oral administration is preferred.

Examples of dosage forms suitable for oral administration include a tablet, a capsule, a powder, a drinkable preparation, a granule, a parvule, a syrup, a solution, an emulsion, a suspension, a chewable tablet, and the like. Examples of dosage forms suitable for parenteral administration include, but are not limited to, an injection (e.g. a subcutaneous, intramuscular or intravenous injection, and the like), a drop, an inhalant, a nebula, a suppository, a gel, ointment or the like.

Liquid formulations suitable for oral administration such as a solution, emulsion or syrup can be produced using water; sugars such as sucrose, sorbit or fructose; glycols such as polyethylene glycol or propylene glycol; oils such as sesame oil, olive oil or soybean oil; antiseptics such as p-hydroxybenzoate; and flavors such as strawberry flavor and peppermint. In order to produce solid formulations such as capsule, tablet, powder or granule, there can be used an excipient such as lactose, glucose, sucrose or mannite; a disintegrator such as starch or sodium alginate; a lubricant such as magnesium stearate or talc; a binder such as polyvinyl alcohol, hydroxypropylcellulose or gelatin; a surfactant such as fatty acid ester; and a plasticizer such as glycerine, and the like.

Formulations for an injection or drop that are suitable for parenteral administration preferably comprise, as an active ingredient, the above substance in a dissolved or suspended state in a sterilized aqueous medium which is isotonic to the recipient's blood. For example, in the case of an injection, a solution can be prepared using an aqueous medium consisting of a saline solution, a glucose solution or a mixture of a saline solution and a glucose solution. In the case of a formulation for intestinal administration, it can be prepared using carriers such as theobroma oil, hydrogenated lipids or hydrogenated carboxylic acid, and can be provided as a suppository. In order to produce a nebula, the above substance as an active ingredient may be dispersed as microparticles, and a carrier which does not irritate the recipient's cavitas oris and respiratory tract mucosa and which facilitates absorption of the active ingredient can be used. Specific examples of carriers include lactose, glycerine, and the like. Formulations having a form such as aerosol or dry powder may be prepared depending on the properties of the substance of an active ingredient and the carrier to be used. One or two or more auxiliary ingredients selected from glycols, oils, flavors, an antiseptic, an excipient, a disintegrator, a lubricant, a binder, a surfactant, a plasticizer and the like may be added to these formulations for parenteral administration.

The agent for enhancing stamina and the agent for promoting glycogen accumulation of the present invention can be mixed into a drinkable preparation, such as a nutrition supplement drinkable preparation, or can be mixed into health food as a food additive. Specific examples of products comprising the agent for enhancing stamina or the agent for promoting glycogen accumulation of the present invention include not only a medicament, but also health foods or supplements including drinks, such as those generally called a soft drink, drinkable preparation, health food, specified supplement food, functional food, function-activating food, nutritional supplementary food, supplement, feed, feed additive and the like.

The dose and dosage frequency of the agent for enhancing stamina and the agent for promoting glycogen accumulation of the present invention are determined as appropriate, depending on various factors such as dosage purpose, dosage form, age, body weight or health condition of a subject, and the exercise load when a subject is an athlete. Generally, the dose of an active ingredient per day is 10 to 2,000 mg/kg, preferably 10 to 200 m/kg, and more preferably 50 to 150 mg/kg. It is preferred that the above dose of formulation is dividedly applied about once to 4 times per day, preferably about twice to 4 times per day.

An administration time of the agent for enhancing stamina and the agent for promoting glycogen accumulation of the present invention is not particularly limited. For example, for an athlete, the agent of the present invention can be administered at any time including before, during, and after exercise, and over any period.

The agent for enhancing stamina and the agent for promoting glycogen accumulation of the present invention can be administered to any mammal including humans, preferably to humans, edible animals (for example, cultured seafood, or livestock animals, such as a pig, cow or chicken), racehorses, dogs for a dogsled, fighting dogs and the like.

The present invention further relates to a supplement comprising a mixture of cyclic and/or straight chain poly lactic acids having a condensation degree of 3 to 20. That is, the mixture of cyclic and/or straight chain poly lactic acids having a condensation degree of 3 to 20 used in the present invention can be used not only by administering in the above isolated dosage form, but also by mixing into foods and drinks. Specific examples of foods and drinks into which the mixture of poly lactic acids can be mixed include confectionary, such as a chewing gum, chocolate, candy, sweet tablet, jelly, cookie, biscuit, and yogurt; frozen deserts, such as ice cream and sherbet; beverages, such as tea, soft drink (including juice, coffee, cocoa and the like), nutrition supplement drinkable preparation, and cosmetic drinkable preparation; and all other foods and drinks, such as bread, ham, soup, jam, spaghetti, and frozen food. Alternatively, the mixture of poly lactic acids used in the present invention can also be used by adding to seasoning, food additives, and the like. By the use of the above foods and drinks (supplements) of the present invention, there can be provided safe foods and drinks which can exert the effect of enhancing stamina while substantially showing no toxic side effect.

The supplement of the present invention encompasses foods and drinks in every form, and the types are not specifically limited. That is, the supplement of the present invention can be provided as a supplement which is prepared by mixing the agent for enhancing stamina or the agent for promoting glycogen accumulation of the present invention into the above-mentioned various foods and drinks, or various nutrient compositions, such as various oral or enteral nutrient preparations or drinks. Compositions of such a supplement may include protein, lipid, carbohydrate, vitamin and/or mineral, in addition to the mixture of cyclic and/or straight chain poly lactic acids having a condensation degree of 3 to 20. The form of the supplement is not specifically limited, and may be in any form, such as solid, powdery, liquid, gel, and slurry forms, so far as it is in a form that is easily ingested.

The content of the mixture of poly lactic acids in a supplement is not specifically limited, and is generally 0.1 to 20 weight %, more preferably approximately 0.1 to 10 weight %.

The mixture of poly lactic acids is preferably contained in a supplement in an amount which facilitates improvement of endurance and stamina upon exercise, which are the purposes of the present invention. Preferably, about 0.1 g to 10 g, more preferably about 0.5 g to 3 g, of the mixture of poly lactic acids is contained per food or drink ingested before or upon exercise.

The present invention is further described in the following examples, but the scope of the present invention is not limited by the examples in any way.

### EXAMPLES

### Production Example 1: Production of a mixture of poly lactic acids (hereinafter also referred to as CPL)

500 ml of L-lactic acid (to which D-lactic acid was also mixed) was placed into a separable flask in a mantle heater. 300ml/min of nitrogen gas was flowed therein while stirring. Accumulated water was introduced into a flask equipped with a reflux condenser via a warmed descending type connecting tube, while heating at 145°C for 3 hours. Furthermore, after pressure was reduced to 150 mmHg and heated at the same temperature for 3 hours, the mixture was heated at 155°C for 3 hours under a reduced pressure of 3 mmHg, and then at 185°C for 1.5 hours under a reduced pressure of 3 mmHg to obtain poly lactic acids as a reaction product.

The obtained poly lactic acids were kept at 100°C, and 100ml of ethanol and 400ml of methanol were separately added thereto, and then the mixture was allowed to be cooled. This mixture was added to 500ml of methanol, and the mixture was well stirred and left to stand. Then, the mixture was filtrated for purification. The filtrate was subjected to vacuum drying and then dissolved in acetonitrile to obtain 200ml (stock solution) in total.

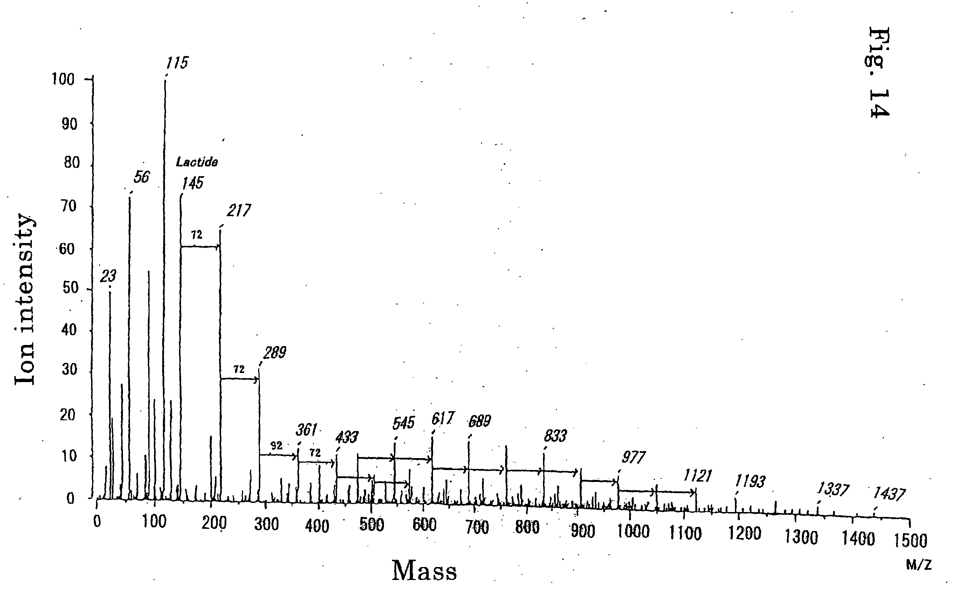

The stock solution was subjected to a reverse phase ODS column (TSK gel ODS-80 TM) which was previously equilibrated, and was stepwise eluted with 30%, 50% and 100% acetonitrile (pH2.0) each containing 0.01M hydrochloric acid to obtain poly lactic acids (condensation degree of 3 to 20) as an acetonitrile 100% elution fraction. The mass spectrum of the obtained substance is shown in Fig. 14. As is clear from the regular fragment ion peaks in Fig. 14, the obtained mixture of poly lactic acids mainly comprises cyclic condensate, and a small amount of linear condensate is contained therein.

### Test Example 1:

### (Test method)

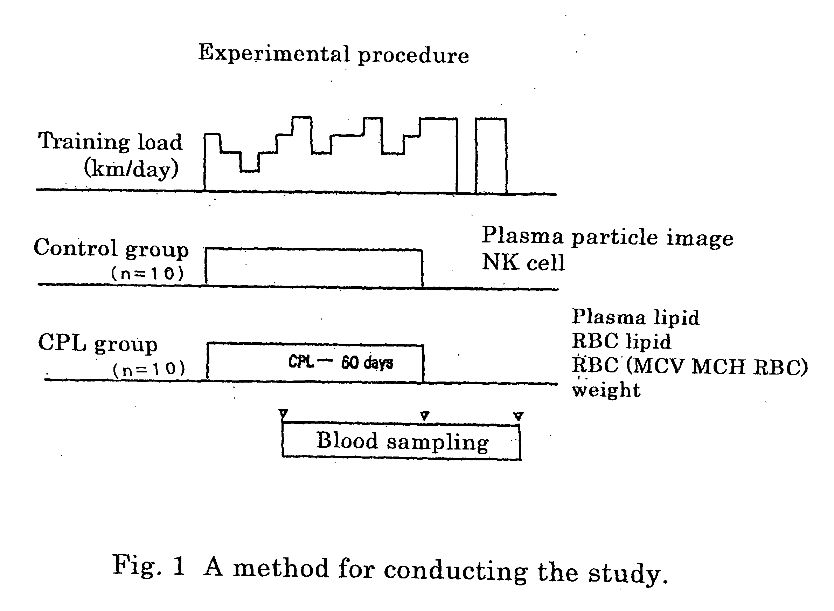

A method for carrying out the test is shown in Fig. 1. Subjects were divided into a group administered with CPL (n=10) and a control group (n=10). Training load, weight fluctuation, blood test, analysis and determination of serum lipid, number of NK cells, and plasma particle images were studied mainly for 60 days of a summer camp and the following 30 days.

The CPL-administered group was ingested with 10g of CPL per day for 60 days.

### (Results)

### 1. Weight fluctuation in respect of training load in the control group and in the CPL-administered group

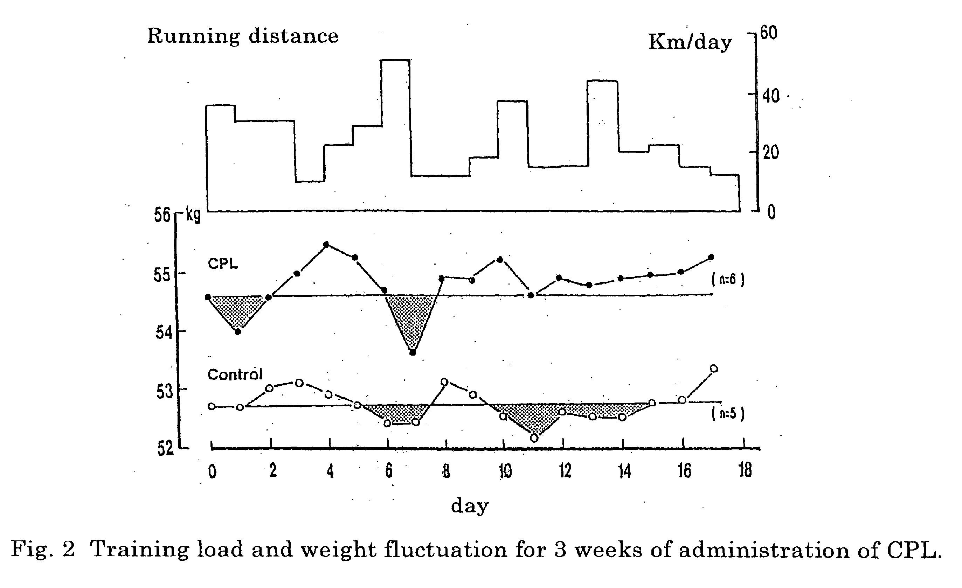

Training load (kg/day) and weight fluctuation in each group for the first 3 weeks are shown in Fig. 2. Less average weight reduction was found in the CPL-administered group as compared with the control group. In particular, this tendency was strongly observed in the latter half of the camp period .

### 2. Difference in diurnal weight fluctuation

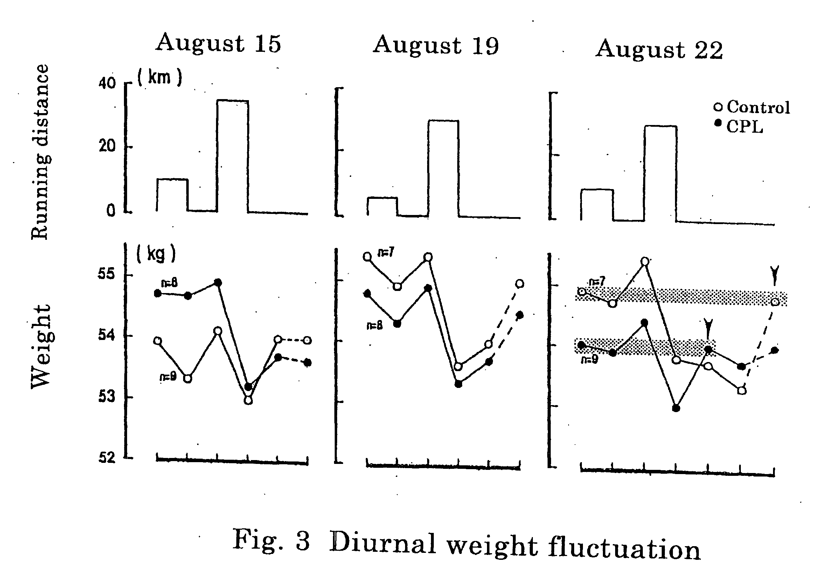

Fig. 3 shows diurnal weight fluctuation for days with a heavy training load (30 to 40 km). The effect of training on the respective day is shown by measurement of body weight conducted a total of 6 times for the day, in early morning, after morning training, before the main training, after the main training, before supper and the next day. By the end of the camp, a difference was observed between the CPL-administered group and the control group. In the control group, weight recovery was delayed till the next day, while in the CPL-administered group, weight recovery was found to be faster.

These results show that training endurance is strongly manifested by administration of CPL.

### 3. Blood test results

The results of blood tests conducted before and after training over the first 3 weeks are shown in Table 1. The results of the blood tests conducted before and after training showed no significant differences between the two groups in any of the measured values.

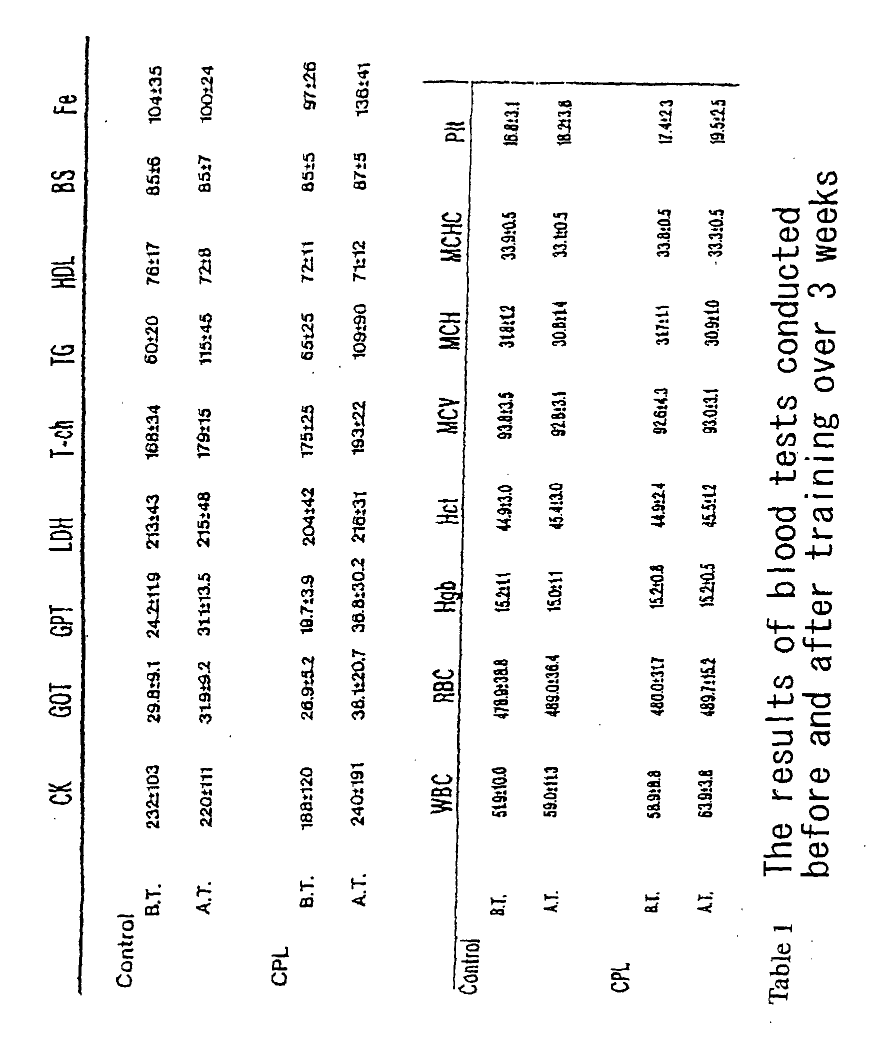

### 4. Effect on plasma lipid

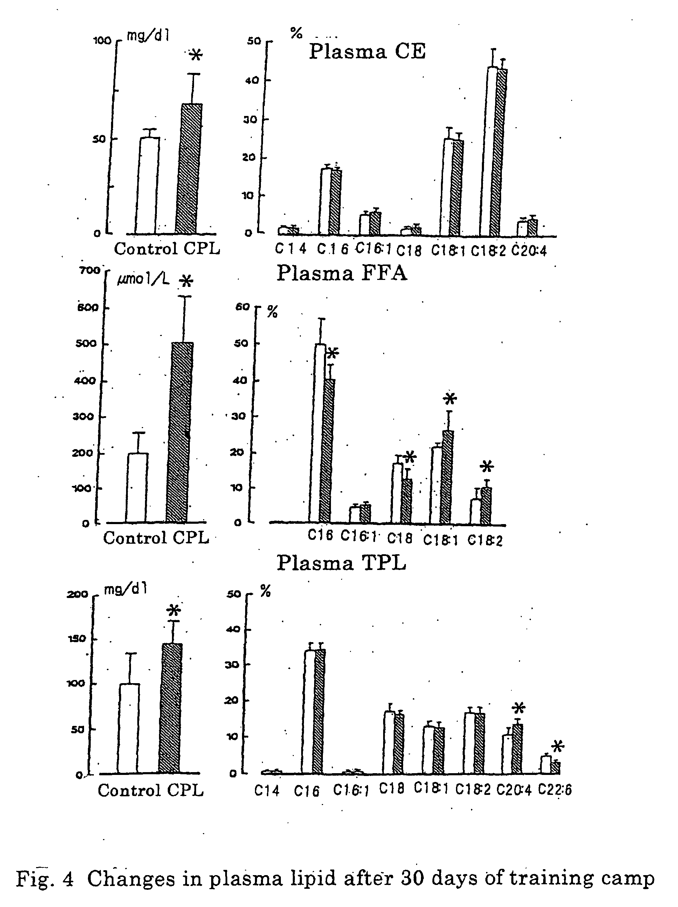

The results of plasma lipid measurements conducted 30 days after the camp are shown in Fig. 4. In the CPL-administered group, plasma FFA was extremely elevated, and plasma CE and total phospholipid (TPL) were also increased. However, there was no increase in plasma TG.

### 5. Changes in erythrocyte lipid

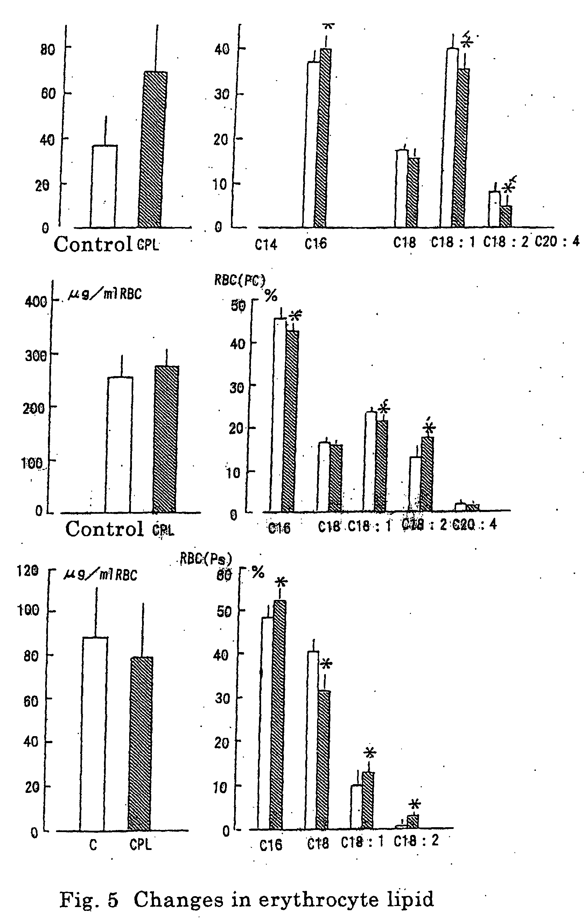

Every 1 month after the end of the camp period, erythrocyte lipid was analyzed and determined (Fig. 5). Results are shown as 1 ml of erythrocyte lipid. For PE (phosphatidylethanol amine), PC (phosphatidylcholine) and PS (phosphatidylserine), significant changes in the ratio of fatty acid composition were respectively found in the CPL-administered group. For quantitatively abundunt PC, an increase was found in linoleic acid.

### 6. Plasma particle image

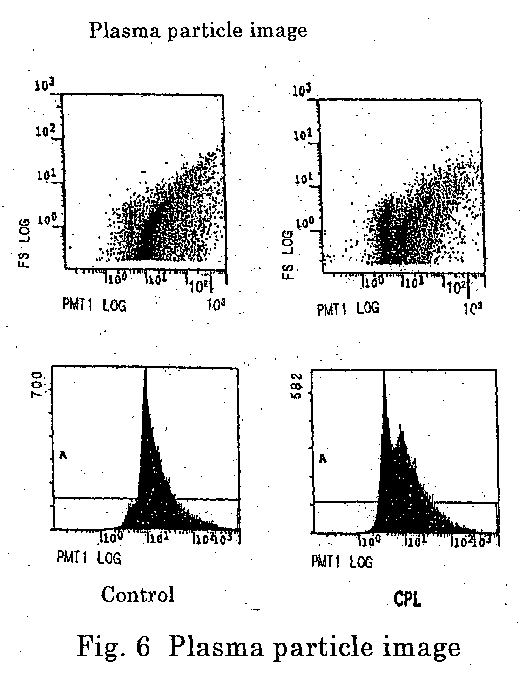

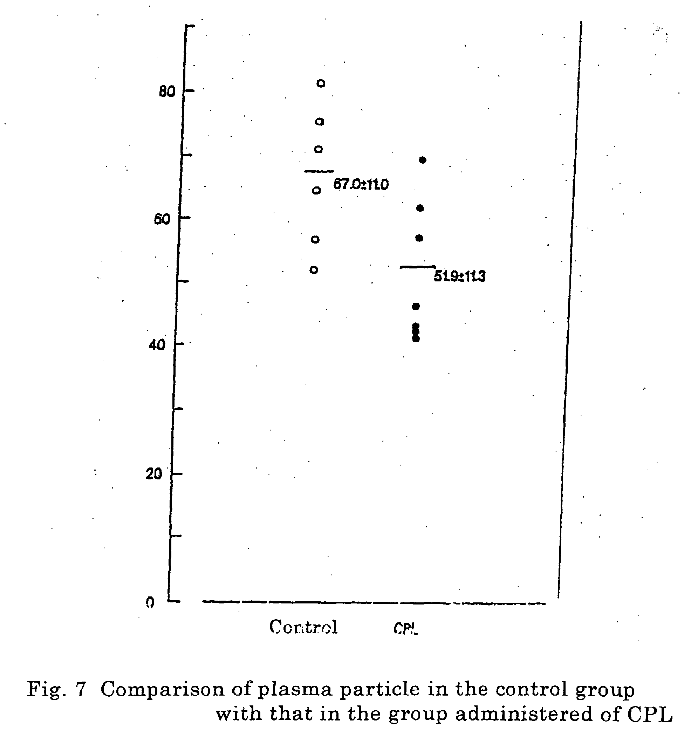

Plasma particle image was produced using ALTRA-EPICS (Fig. 6). As shown in a typical example, many small particles emerged in the plasma of the CPL-administered group. The results are summarized in Fig. 7. It is also statistically understood that plasma particles became smaller as a whole due to administration of CPL.

### 7. NK cell

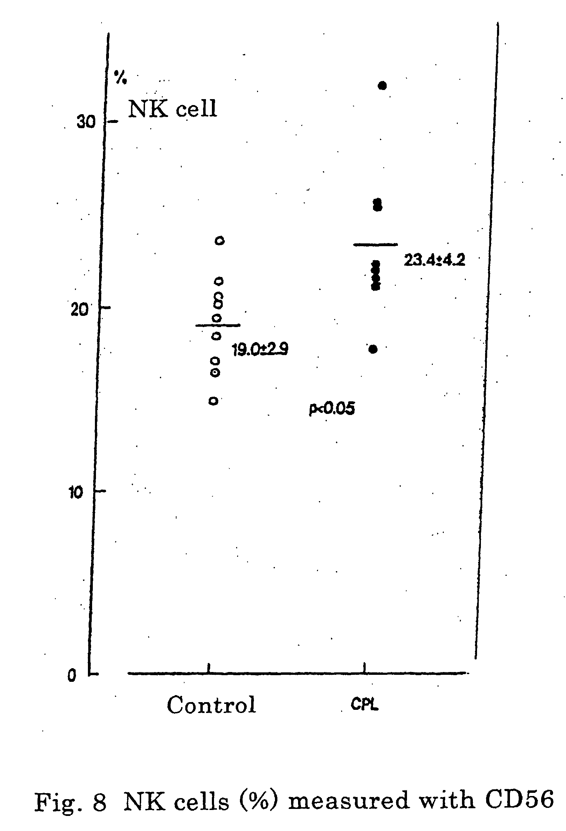

The NK cells (%) measured using CD56 was indicated by ALTRA-EPICS. Figure 8 shows the results. A significant increase due to administration of CPL was found in the number of NK cells.

### 8. Improvement of personal records in 10,000-meter race

After the two months of camp, a 10,000-meter race was held in which the athletes times were recorded. This is a method for testing endurance against training and improvement in endurance ability.

In the control group, 3 out of 10 runners set new personal records, while in the CPL-administered group, 6 out of 9 runners set new personal records. Approximately twice as many runners in the CPL-administered group set new personal records compared to the control group.

### 9. Summary

The above results are summarized as follows.
(1) Less weight reduction and rapid weight recovery were observed in the CPL-administered group.
(2) Regarding plasma lipids, significant increases were found in plasma CE, FFA, and TPL in the CPL-administered group.
(3) Regarding erythrocyte lipid, an increase was found in PE (phosphatidylethanol amine).
(4) A significant change was observed in the plasma particle image.
(5) The NK cells (measured with CD56) was increased by administration of CPL.
(6) Twice the number of runners in the CPL-administered group set new personal records in a 10,000-meter race.

These results suggest that administration of CPL has an influence not only on body weight and fat tissue, but also on the entire stress responding system of the organism. Further, administration of CPL is effective as a supplement or the like, since it enhances durability to training.

### Test example 2:

### (Method)

The subjects were 3 healthy adult men (average age of 45). Endurance exercise using the blood lactic acid level of 4 mM as an index was employed for the intensity of exercise load. In the method for determining the intensity of the load at 4 mM level, 4 to 5 types of different rates were selected according to the exercise ability of individual subjects using a treadmill (the angle of inclination was fixed at 8%). The subjects were made to run at each of the rates in ascending order for respective exercise periods consisting of 6 min. Between each exercise period, a 6-minute interval was given. Blood was collected in minute quantities from the fingertip immediately after every exercise of 6 min to measure blood lactic acid level. The 4 mM level of blood lactic acid was determined by plotting the blood lactic acid level relative to treadmill rate, and then calculating by an interpolation method a rate at which the lactic acid level corresponds to 4 mmol/l. In this test, 20 min of endurance running at a treadmill rate corresponding to the 4mM level was separately loaded on day 12, 20, and 40 after administration of CPL, and the changes in energy metabolism were compared and studied. Environmental conditions upon exercise were regulated to maintain an atmosphere pressure of 760 mmHg, room temperature of 20°C, and relative humidity of 55%. The dose of CPL per day was 10 g.

Regarding the measurement items and measurement methods, the blood lactic acid level was measured using a glucose lactate analyzer 2300STAT (YSI); and the amount of energy metabolism was measured using K4 telemetry system (Cosmed) for measuring respiratory metabolism.

### (Results)

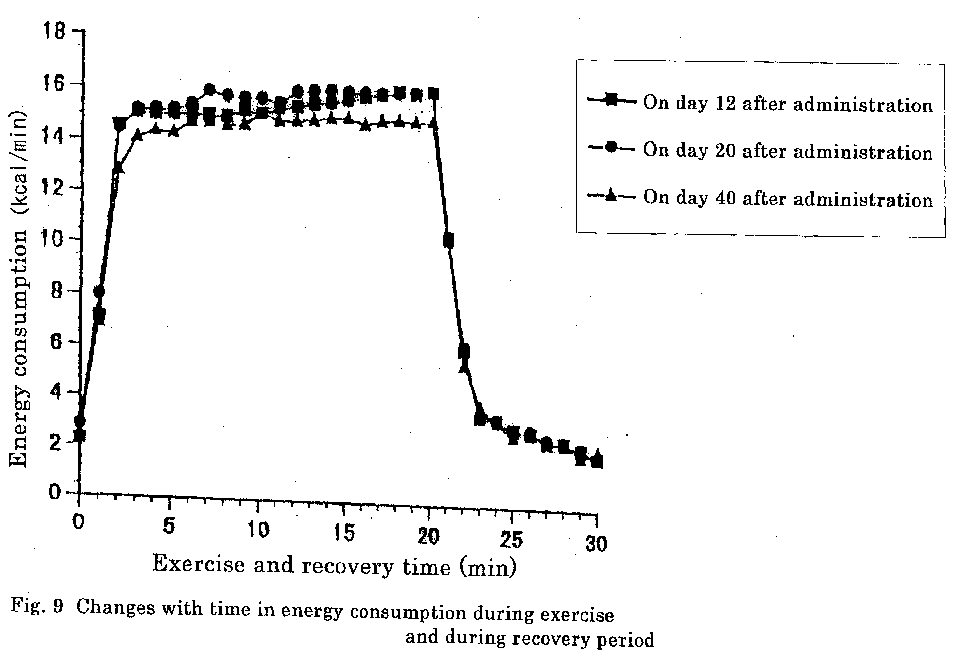

Regarding changes with time (Fig. 9) in energy consumption during exercises and recovery periods (10 min) under each administration condition, energy consumptions during exercise were lower on day 40 after administration of CPL than on day 12 and day 20 after administration of CPL. Total energy consumptions during exercise were 300 kcal on day 12, 306 kcal on day 20, and 286 kcal on day 40 after administration of CPL.

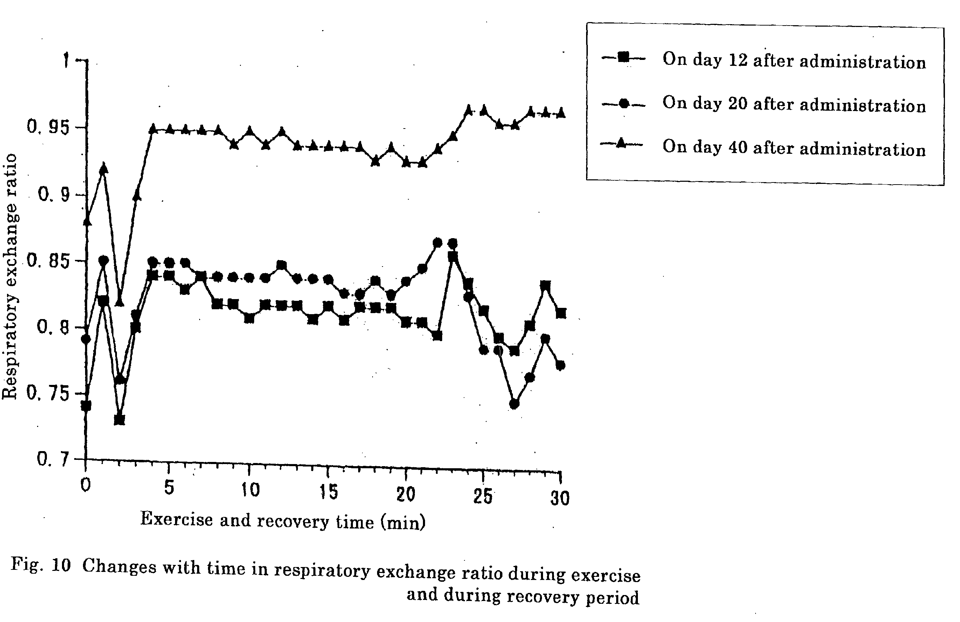

Regarding changes with time in respiratory exchange ratio during exercise and in recovery period under each condition shown in Fig. 10, the respiratory exchange ratio on day 40 after administration both during exercise and in recovery period was maintained higher than those on day 12 and 20 after administration. Energy consumptions from fat and carbohydrate that were deduced from the respiratory exchange ratio were, during exercise, 188 kcal of fat and 112 kcal of carbohydrate on day 12 after administration and 172 kcal of fat and 135 kcal of carbohydrate on day 20 after administration. On day 40 after administration, the energy consumption of fat had decreased to 66 kcal, while that of carbohydrate had elevated to 220 kcal.

As described above, in Test example 2, a significant increase in the respiration exchange ratio during exercise was found on day 40 after administration of CPL as compared to the respiration exchange ratios during exercise on day 12 and 20 after administration of CPL. These results suggest the possibility that if administration of CPL is continued for a long period to a human without an exercise habit, energy for exercise will come to depend mainly on carbohydrate metabolism.

### Test example 3:

### (Method)

The subjects were 3 oarsmen (average age of 19) who routinely train hard to compete in boat races, which are competitions in which a participant's energy mainly depends on carbohydrate metabolism. As the method of exercise loading, a rowing ergometer was used before administration of CPL and on day 14 and day 30 after administration of CPL, in a way which involved, similar to Test example 2, 20 min of exercise using the blood lactic acid level as an index, a 10-minute interval, and then a 1,000 m time trial. Then, the energy metabolisms and the performance test were compared and studied (for energy metabolisms, the energy metabolisms before and on day 14 after administration of CPL were compared). The dose of CPL per day was 6 g.

### (Results)

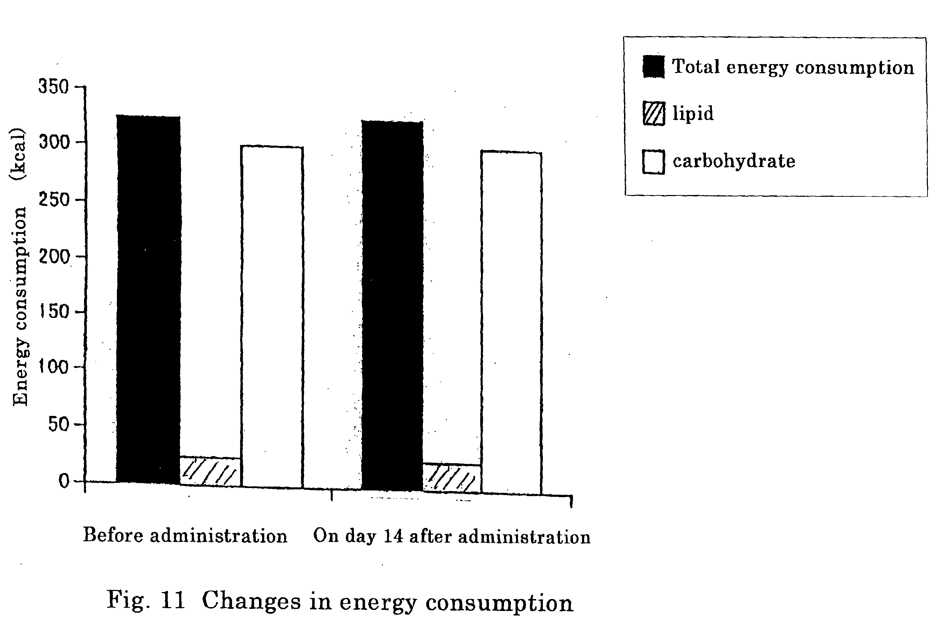

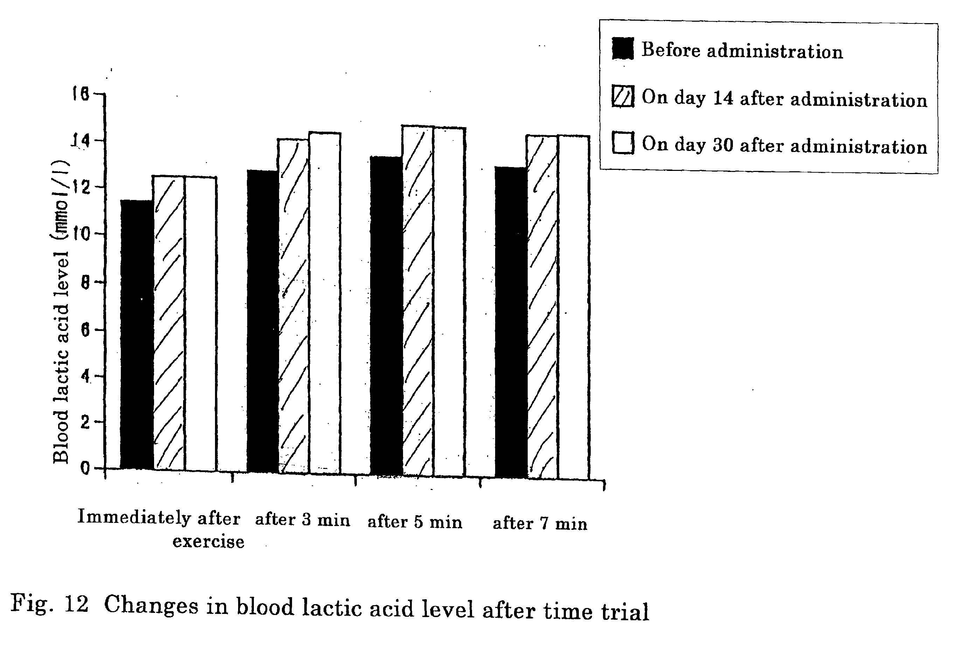

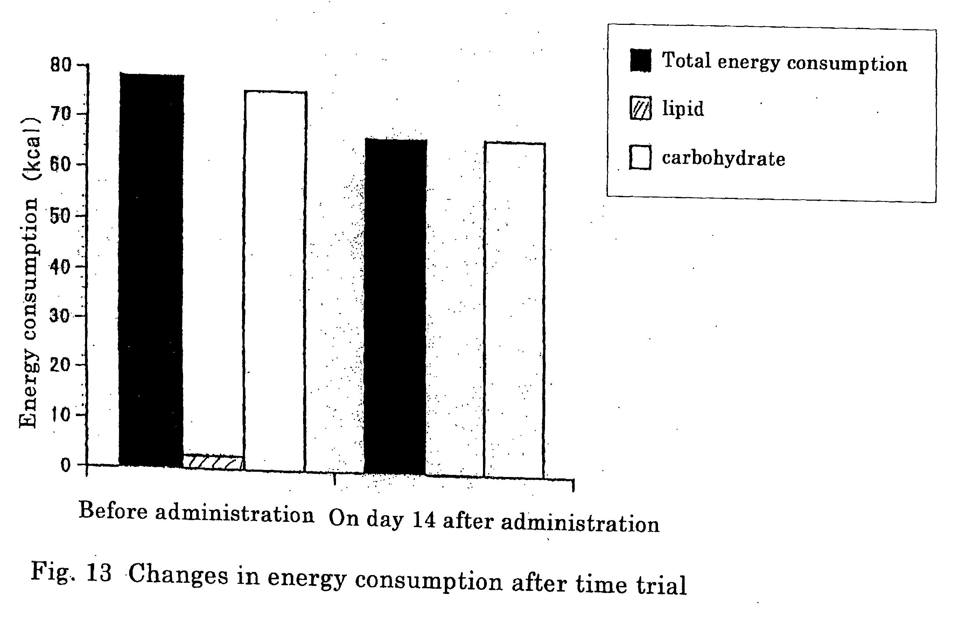

The total energy consumption during exercise (20 min), for which the blood lactic acid level shown in Fig. 11 was used as an index, and energy consumptions from carbohydrate and fat were almost the same before administration of CPL and on day 14 after administration of CPL. Next, the time recorded in a 1,000 m time trial after an interval was 3 min and 26.5 sec before administration, whereas after administration with CPL this was reduced significantly to 3 min and 23.6 sec on day 14 and 3 min and 20.7 sec on day 30. The blood lactic acid levels (Fig. 12) after time trial on day 14 and day 30 after administration of CPL were both higher than that before administration. As shown in Fig. 13, the total energy consumption on day 14 after administration of CPL (67 kcal) was lower than that before administration (78 kcal).

As described above, in the case of an athlete who routinely trains hard for a competition in which a participant's energy mainly depends on carbohydrate metabolism, administration of CPL enables more efficient use of energy while improving anaerobic and aerobic metabolisms of carbohydrate, suggesting a possibility of enabling improvement in performance.

### Production Example 1

A THF solution (2ml) in which 0.033g (1.03mmol) of methanol was dissolved was added to a 50ml double-cap eggplant-shaped flask under a nitrogen atmosphere, and cooled to -78°C in a dry ice/acetone bath. Then, 0.64ml (1.00mmol) of n-butyl lithium was added thereto and the mixture was stirred for 15 minutes. Further, a THF solution (2ml) in which 0.576g (4.00mmol) of (3R,6R)-(+)-3,6-dimethyl-1,4-dioxane-2,5-dione was dissolved was added thereto and stirred, and the temperature was gradually raised to room temperature over 4 hours.

After completion of stirring, 2ml of saturated ammonium chloride was added to the mixture while maintaining a nitrogen atmosphere, and 10ml of water was further added thereto. The mixture was extracted with chloroform and a saturated saline solution and washed, and then anhydrous sodium sulfate was added thereto and dried overnight. The obtained product was subjected to vacuum concentration in which solvent was completely removed with a vacuum pump. As a result, 0.551g (yield 90.5%) of product consisting of a mixture of cyclic oligo-lactate and chain oligo-lactate was obtained with a weight ratio between cyclic oligomer and chain oligomer being 84 : 16.

### Production Example 3

A THF solution (2ml) in which 0.054g (1.17mmol) of ethanol was dissolved was added to a 50ml double-cap eggplant-shaped flask under a nitrogen atmosphere, and cooled to -78°C in a dry ice/acetone bath. Then, 0.64ml (1.00mmol) of n-butyl lithium was added thereto and the mixture was stirred for 15 minutes. Further, a THF solution (2ml) in which 0.576g (4.00mmol) of (3R,6R)-(+)-3,6-dimethyl-1,4-dioxane-2,5-dione was dissolved was added thereto and stirred for 30 minutes.

After completion of stirring, 2ml of saturated ammonium chloride was added to the mixture while maintaining a nitrogen atmosphere and 10ml of water was further added thereto, and then the temperature was raised to room temperature by removal of the dry ice/acetone bath. Subsequently, the mixture was extracted with 20ml of ether 8 times, and the ether layer was washed with 30ml of saturated saline solution. Then, anhydrous sodium sulfate was added thereto and dried while stirring for 1 hour. The obtained product was subjected to vacuum concentration in which solvent was completely removed with a vacuum pump. As a result, 0.535g (yield 84.9%) of product consisting of a mixture of cyclic oligo-lactate and chain oligo-lactate was obtained with a weight ratio between cyclic oligomer and chain oligomer being 82:18.

### Production Example 4

A THF solution (2ml) in which 0.062g (1.03mmol) of 2-propanol was dissolved was added to a 50ml double-cap eggplant-shaped flask under a nitrogen atmosphere, and cooled to -78°C in a dry ice/acetone bath. Then, 0.64ml (1.00mmol) of n-butyl lithium was added thereto and the mixture was stirred for 15 minutes. Further, a THF solution (2ml) in which 0.576g (4.00mmol) of (3R,6R)-(+)-3,6-dimethyl-1,4-dioxane-2,5-dione was dissolved was added thereto and stirred, and the temperature was gradually raised to room temperature over 4 hours.

After completion of stirring, 2ml of saturated ammonium chloride was added to the mixture while maintaining a nitrogen atmosphere, and 10ml of water was further added thereto. The mixture was extracted with chloroform and a saturated saline solution and washed, and then anhydrous sodium sulfate was added thereto and dried overnight. The obtained product was subjected to vacuum concentration in which solvent was completely removed with a vacuum pump. As a result, 0.589g (yield 92.3%) of product consisting of a mixture of cyclic oligo-lactate and chain oligo-lactate was obtained with a weight ratio between cyclic oligomer and chain oligomer being 80 : 20.

### Production Example 5

A THF solution (2ml) in which 0.074g (1.00mmol) of tert-butanol was dissolved was added to a 25ml double-cap eggplant-shaped flask under a nitrogen atmosphere, and cooled to -78°C in a dry ice/acetone bath. Then, 0.64ml (1.00mmol) of n-butyl lithium was added thereto and the mixture was stirred for 15 minutes. Further, a THF solution (2ml) in which 0.434g (3.01mmol) of (3R,6R)-(+)-3,6-dimethyl-1,4-dioxane-2,5-dione was dissolved was added thereto and stirred, and the temperature was gradually raised to room temperature over 2.5 hours.

After completion of stirring, 2ml of saturated ammonium chloride was added to the mixture while maintaining a nitrogen atmosphere, and 10ml of water was further added thereto. The mixture was extracted with chloroform and a saturated saline solution and washed, and then anhydrous sodium sulfate was added thereto and dried overnight. The obtained product was subjected to vacuum concentration in which solvent was completely removed with a vacuum pump. As a result, 0.537g (yield 82.5%) of cyclic oligo-lactate wherein all asymmetric carbon atoms have an R configuration, was obtained. [α] = +125.1° . mp=132.5-132.4°C

### Production Example 6

A THF solution (2ml) in which 0.117g (1.06mmol) of thiophenol was dissolved was added to a 50ml double-cap eggplant-shaped flask under a nitrogen atmosphere, and cooled to -78°C in a dry ice/acetone bath. Then, 0.64ml (1.00mmol) of n-butyl lithium was added thereto and the mixture was stirred for 15 minutes. Further, a THF solution (2ml) in which 0.576g (4.00mmol) of (3R,6R)-(+)-3,6-dimethyl-1,4-dioxane-2,5-dione was dissolved was added thereto and stirred, and the temperature was gradually raised to room temperature over 4 hours.

After completion of stirring, 2ml of saturated ammonium chloride was added to the mixture while maintaining a nitrogen atmosphere, and 10ml of water was further added thereto. The mixture was extracted with chloroform and a saturated saline solution and washed, and then anhydrous sodium sulfate was added thereto and dried overnight. The obtained product was subjected to vacuum concentration in which solvent was completely removed with a vacuum pump. As a result, 0.612g (yield 88.3%) of product was obtained. It was confirmed by NMR analysis that this product comprised cyclic oligo-lactate and chain oligo-lactate at a weight ratio of 96 : 4.

0.238g of the product was isolated and purified using silica gel chromatography (solvent; hexane : ether = 1 : 2) to obtain 5 fractions (fraction Nos. 10-1 to 10-5).

### Production Example 7

3ml of THF solution containing 0.089g (1mmol) of S-lactic acid amide was added to a 50ml double-cap eggplant-shaped flask at room temperature under a nitrogen atmosphere, and 0.64ml (1.00mmol) of n-butyl lithium was reacted therewith at -78°C followed by stirring for 15 minutes. Further, 2ml of THF solution containing 0.576g (4mmol) of L-(-)-lactide was added thereto and reacted therewith for 30 minutes, and then the temperature was raised from -78°C to 0°C followed by reaction for 1.5 hours. Subsequently, the temperature was further raised to room temperature by addition of 5ml of saturated ammonium chloride solution. After the mixture was extracted with chloroform, the organic layer was washed with a saturated saline solution, and dried with anhydrous sodium sulfate followed by vacuum concentration (NMR sa0140), and the residue was separated into 3 fractions by silica gel chromatography (solvent; ether: hexane = 2:1).

### Test example 4

### (1) Experimental method

Wister rats (body weight: 150g, male) were divided into 3 groups (6 rats in each group) A, B and C. Group A was fed with CE-2 standard solid food (CLEA Japan, Inc.), and groups B and C were fed with special food containing the agent for promoting glycogen accumulation. Drinking water was provided ad libitium. Group C was loaded with exercise comprising a daily swimming of 10 min for the first week after the start of feeding, and a daily swimming of 20 min for the next 1 week, followed by a daily swimming of 30 min for the remaining period.

The special food containing the agent for promoting glycogen accumulation was prepared by consignment to CLEA Japan, Inc. With the exception that the special food contained 1 weight % of the agent for promoting glycogen accumulation (obtained in production example 2), all the other nutrient components thereof were the same as those of CE2 standard solid food.

Following half a day of fasting on day 32 after the start of feeding, all the animals were euthenised under ether anesthesia. After removal of blood, the muscle was removed and the glycogen content of the muscle was quantitatively analyzed. The experimental results are indicated with mean value ± standard deviation. Student t-test was used for significance test.

### (2) Experimental results

The obtained results are shown in Table 2 below.

**Table 2:**

| Effect of the agent for promoting glycogen accumulation on the glycogen level in the muscle | | |
|---|---|---|
| Group (n) | Glycogen level (mg/g tissue wet weight) | |
| | Soleus muscle | Plantar muscle |
| A (n = 6) | 1.72 ± 0.47 | 5.28 ± 0.74 |
| B (n = 6) | 2.09 ± 0.38 | 6.38 ± 0.98 ¹⁾ |
| C (n = 6) | 2.36 ± 0.56 ¹⁾ | 7.08 ± 1.38 ^{2),3)} |

| | | |
|---|---|---|
| 1) Significantly different compared to group A (P<0.05) | | |
| 2) Significantly different compared to group A (P<0.01) 3) Significantly different compared to group B (P<0.05) | | |

As is clear from the results in Table 2, the glycogen content of the plantar muscle was significantly increased (P<0.05) by feeding the rats with the special food containing the agent for promoting glycogen accumulation of the present invention. The combination of feeding with the special food and swimming significantly increased the glycogen contents of both the soleus muscle and the plantar muscle (P<0.05, P<0.01).

### Test example 5

### (1) Experimental method

ICR mice (body weight: 10g, male) were divided into 2 groups (6 mice in each group) D and E. Group D was fed with CE-2 standard solid food, and group E was fed with special food (which was the same as that used in test example 4) containing the agent for promoting glycogen accumulation. Drinking water was provided ad libitium.

On day 14 after the start of feeding, the animals were euthenised under ether anesthesia. After removal of blood, the liver was removed and the glycogen content thereof was quantitatively analyzed. The experimental results are indicated with mean value ± standard deviation. Student t-test was used for significance test.

### (2) Experimental results

The obtained results are shown in Table 3 below.

**Table 3:**

| Effect of the agent for promoting glycogen accumulation on the glycogen level in the liver | |
|---|---|
| Group (n) | Glycogen level in the liver (mg/g tissue wet weight) |
| D(n=6) | 24.9 ± 11.2 65.7 ± 7.19¹⁾ |
| E(n=6) | |

| | |
|---|---|
| 1) Significantly different compared to group D (P<0.001) | |

As is clear from the results in Table 3, the amount of glycogen accumulation in the mouse liver was significantly increased (P<0.001).

### (Evaluation of experimental results)

The results of test examples 4 and 5 show that feeding with the special food containing the agent for promoting glycogen accumulation of the present invention increased the amounts of glycogen accumulation in the liver and the muscle, and a combination of the special food and exercise further increased the glycogen content in the muscle. The agent for promoting glycogen accumulation of the present invention significantly promoted glycogen accumulation for two types of animals of different species, rat and mouse, belonging to the rodent. Therefore, it can be expected that ingestion of the agent for promoting glycogen accumulation of the present invention also increases the amounts of glycogen accumulation in the muscle and liver of humans.

### INDUSTRIAL APPLICABILITY

The agent for enhancing stamina of the present invention can be used, for example, for maintaining or improving the endurance of athletes, and can be used to recover from fatigue. Administration with the agent for enhancing stamina of the present invention to athletes enhances their endurance of training. Thus, the agent for enhancing stamina of the present invention is effective as, for example, a supplementary means for improving competitive ability. Moreover, the agent for promoting glycogen accumulation of the present invention is useful for recovering from fatigue, enhancing the exercise ability of the muscle, or improving the QOL of patients, and is also useful in improving carnosity. The agent for promoting glycogen accumulation of the present invention can exert an excellent effect for these purposes. Further, since the mixture of poly lactic acids which is used as an active ingredient in the present invention is a low condensed form of lactic acid derived from a biological ingredient, it has advantages including a high biocompatibility, low side effects, and a relatively low costing raw material.

## Claims

1. An agent for enhancing stamina which comprises a mixture of cyclic and/or straight chain poly lactic acids having a condensation degree of 3 to 20.

2. The agent for enhancing stamina according to claim 1 which is used to maintain or improve the endurance of athletes.

3. The agent for enhancing stamina according to claim 1 which is used to recover from fatigue.

4. The agent for enhancing stamina according to any one of claims 1 to 3 wherein the mixture of cyclic and/or straight chain poly lactic acids having a condensation degree of 3 to 20 comprises a cyclic lactic acid oligomer which is shown by the following formula (1): 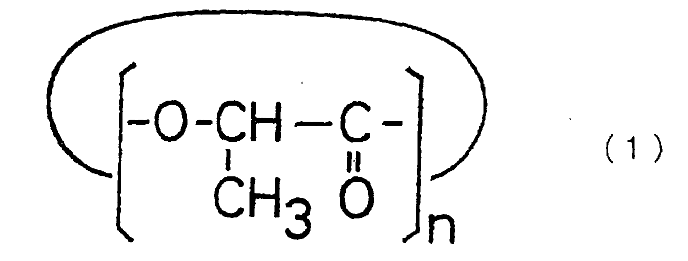 wherein n represents an integer from 3 to 20.

5. The agent for enhancing stamina according to any one of claims 1 to 4, wherein the lactic acid which is a repeating unit in the poly lactic acid, substantially consists of L-lactic acid.

6. The agent for enhancing stamina according to any one of claims 1 to 5 wherein the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fraction of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

7. The agent for enhancing stamina according to claim 6 wherein condensation by dehydration is performed by stepwise decompression and temperature rise under nitrogen gas atmosphere.

8. The agent for enhancing stamina according to claim 6 or 7 wherein reverse phase column chromatography is performed by ODS column chromatography.

9. The agent for enhancing stamina according to any one of claims 1 to 8, which does not substantially comprise a chain lactic acid oligomer having a condensation degree of 3 to 20.

10. A supplement which comprises the agent for enhancing stamina according to any one of claims 1 to 9.

11. An agent for promoting glycogen accumulation which comprises a mixture of cyclic and/or straight chain poly lactic acids having a condensation degree of 3 to 20.

12. The agent for promoting glycogen accumulation according to claim 11 which is used to recover from fatigue, enhance the exercise ability of the muscle or improve the QOL of patients.

13. The agent for promoting glycogen accumulation according to claim 11 which is used for improving camosity.

14. The agent for promoting glycogen accumulation according to any one of claims 11 to 13 wherein the mixture of cyclic and/or straight chain poly lactic acids having a condensation degree of 3 to 20 comprises a cyclic lactic acid oligomer which is shown by the following formula (1): 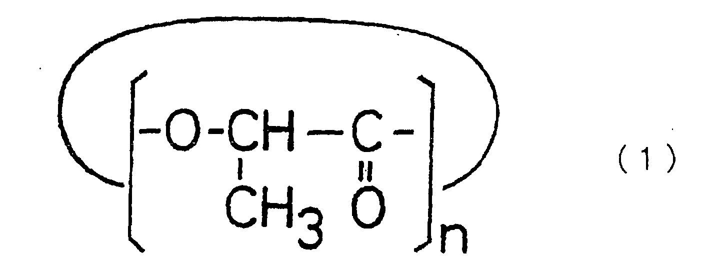 wherein n represents an integer from 3 to 20.

15. The agent for promoting glycogen accumulation according to any one of claims 11 to 14, wherein the lactic acid which is a repeating unit in the poly lactic acid, substantially consists of L-lactic acid.

16. The agent for promoting glycogen accumulation according to any one of claims 1 to 15 wherein the mixture of cyclic and/or straight chain poly lactic acids having a condensation degree of 3 to 20 is a fraction obtained by condensing lactic acids by dehydration under an inactive atmosphere, subjecting the ethanol- and methanol-soluble fraction of the obtained reaction solution to reverse phase column chromatography, and eluting with 25 to 50 weight % acetonitrile aqueous solution of pH 2 to 3 and then with 90 weight % or more acetonitrile aqueous solution of pH 2 to 3.

17. The agent for promoting glycogen accumulation according to claim 16 wherein condensation by dehydration is performed by stepwise decompression and temperature rise under nitrogen gas atmosphere.

18. The agent for promoting glycogen accumulation according to claim 16 or 17 wherein reverse phase column chromatography is performed by ODS column chromatography.

19. The agent for promoting glycogen accumulation according to any one of claims 11 to 18, which does not substantially comprise a chain lactic acid oligomer having a condensation degree of 3 to 20.

20. A health food or a supplement which comprises the agent for promoting glycogen accumulation according to any one of claims 11 to 19.
